# EUROPÄISCHE PATENTANMELDUNG

(11) **EP 1 008 653 A2**
(43) Veröffentlichungstag der Anmeldung: **14.06.2000**
(21) Anmeldenummer: 99123617.5
(22) Anmeldetag: 26.11.1999
(51) Int. Cl.: C12N 15/861, A61K 48/00

(54) **Rekombinanter, adenoviraler Vektor mit limitierter Autoreplikationsfähigkeit in vivo**

(30) Priorität: 04.12.1998 DE 19856065
(71) Anmelder: Aventis Behring Gesellschaft mit beschränkter Haftung, 35002 Marburg (DE)
(72) Erfinder: Poller, Wolfgang Christian, Dr., 14612 Falkensee (DE)

(57) **Zusammenfassung**

Es wird ein rekombinanter, adenoviraler Vektor beschrieben, der nach teilweiser Deletion der E1-Region noch die Fähigkeit zur Autoreplikation der Vektor-DNA besitzt, aber keine infektiösen Viruspartikel mehr bilden kann. Dieser Vektor kann eine transgene DNA-Sequenz für ein therapeutisch wirksames Gen tragen und in der somatischen Gentherapie eingesetzt werden, wobei die Transgen-Stabilität gegenüber Vektoren ohne die Fähigkeit zur Autoreplikation signifikant erhöht ist.

## Beschreibung

Der Gegenstand der Erfindung ist ein adenoviraler Vektor mit erhöhter Stabilität in vivo, der sich zum Gentransfer bei der somatischen Gentherapie eignet.

Es ist bekannt, daß die meisten gegenwärtig für den Gentransfer in vivo benutzten Vektoren in zwei Klassen eingeteilt werden können. Die erste Klasse von Vektoren bewirkt eine stabile Integration der transgenen DNA-Sequenz in das Zellgenom der Wirtszelle. Hierzu gehören die adeno-assoziierten viralen Vektoren und die retroviralen Vektoren (1), (2), (3). Die zweite Klasse umfaßt Vektoren, die in episomaler Form außerhalb des Genoms der Wirtszelle überleben. Hierzu gehören z.B. die adenoviralen Vektoren (4), (5). Die zur letztgenannten Klasse gehörenden Vektoren haben in vivo nur eine beschränkte Lebensdauer und werden spätestens nach einigen Monaten durch natürlichen Abbau wieder aus der Wirtszelle ausgeschieden.

Für den Erfolg der somatischen Gentherapie chronischer, monogener Krankheiten ist es jedoch von entscheidender Bedeutung, für den Transfer klonierter Gene in vivo einen Vektor zur Verfügung zu stellen, der eine dauerhafte Therapie des Gen-defizienten Patienten durch eine möglichst einmalige oder nur in großen zeitlichen Abständen zu wiederholende Applikation des eine transgene DNA-Sequenz für das fehlende Gen tragenden Vektors gewährleistet.

In den letzten Jahren sind bereits verschiedene Gentransfersysteme mit dem langfristigen Ziel ihrer Anwendung für die somatische Gentherapie entwickelt worden. Eines der vielversprechendsten Systeme sind die sogenannten replikationsdefekten, d.h. nicht-infektiösen adenoviralen Vektoren, die nicht nur ex vivo, sondern auch in vivo im intakten Gesamtorganismus eine sehr hohe Gentransfereffizienz gewährleisten, wie sie bisher mit keinem anderen System erreicht werden konnte. Das adenovirale Vektorsystem hat in seiner bisherigen Form allerdings noch gravierende Mängel. Zum einen ist die Expressionsdauer in vivo in der Regel auf wenige Wochen limitiert, zum anderen löst der Vektor selbst im Empfänger immunologische Reaktionen aus, die nicht nur die Vektorelimination aus dem Zielgewebe beschleunigen, sondern dort auch zu immunpathologischen Phänomenen führen. Falls die obengenannten Probleme des adenoviralen Systems überwunden werden könnten, wäre dies ein wesentlicher Fortschritt auf dem Wege zu einer langfristigen Gentherapie monogener und anderer Krankheiten.

Die Aufgabe der vorliegenden Erfindung ist deshalb die Weiterentwicklung replikationsdefekter, d.h. nicht-infektiöser adenoviraler Vektorsysteme mit dem Ziel der Überwindung der vorstehend genannten Probleme. Vor allem kommt es darauf an, den Transfer von Genen mittels adenoviraler Vektoren so zu verbessern, daß eine gegenüber dem bisherigen Verfahren erheblich verlängerte Transgenstabilität erreicht wird.

Die vorliegende Erfindung beruht nun auf der zentralen Überlegung, daß ein dauerhaftes Verbleiben eines mit einer transgenen DNA-Sequenz beladenen adenoviralen Vektors in seiner Zielzelle dann erreicht werden könnte, wenn es gelingt, eine "Autoreplikation" des Vektorgenoms(DNA) in einem Maße sicher zu stellen, der dem unvermeidlichen natürlichen Abbau des Vektors entspricht, wobei jedoch weiterhin eine Infektiosität des Vektorgenoms ausgeschlossen bleiben muß. Bisher erschien es unmöglich, dieses Ziel mit den bekannten Methoden zur genetischen Veränderung von Adenoviren zu erreichen, weil eine weitestgehende Entfernung der für eine normale Virusvermehrung verantwortlichen E1-Region unerläßlich erschien, um eine Infektion des Wirtsorganismus mit virulenten Adenoviren zu verhindern.

Es wurde nun gefunden, daß überraschenderweise rekombinante, adenovirale Vektoren nach nur teilweiser Deletion der E1-Region noch die Fähigkeit zu einer "Autoreplikation" zurückbehalten.

Unter Autoreplikation wird dabei die limitierte Neusynthese des Vektorgenoms selbst verstanden, ohne daß gleichzeitig eine Synthese relevanter Mengen adenoviraler Strukturproteine erfolgt. Ebenso wie allen E1-deletierten Vektoren fehlt damit auch den zur Autoreplikation befähigten Vektoren die Fähigkeit zur Produktion infektiöser Viruspartikel, jedoch sind die meisten der heute verwendeten E1-Vektoren aufgrund größerer Deletionen im E1-Bereich im Gegensatz zu den erfindungsgemäßen Vektoren nicht zur Autoreplikation ihrer Vektor-DNA befähigt.

Ein erfindungsgemäßer rekombinanter, adenoviraler Vektor, der eine transgene DNA-Sequenz für ein therapeutisch wirksames Gen trägt, ist so strukturiert, daß er zur Autoreplikation der Vektor-DNA in einem Maß befähigt ist, der in etwa dem natürlichen Abbau des Vektors entspricht. Unter anderem läßt sich ein adenoviraler Vektor mit diesen gewünschten Eigenschaften dadurch herstellen, daß seine E1A-Region vollständig entfernt wird, während das E1B-55k Gen zwischen den Nukleotidpositionen 3334 bis 3508 sowie das komplette E1B-14k Gen beginnend mit der Nukleotidposition 3608 erhalten bleiben.

Während bei Adenovektoren unter dem allgemeinen Begriff "Replikation" eine Neusynthese kompletter, infektiöser adenoviraler Partikel in vektorinfizierten Zellen verstanden wird, wird im Sinne der vorliegenden Erfindung als "Autoreplikation" die Neusynthese der Vektor-genomischen DNA verstanden, wobei gleichzeitig eine Synthese anderer viraler Proteine außer denen, die für die Vektor-DNA-Neusynthese benötigt werden, unterbleibt. Der erfindungsgemäße adenovirale Vektor transkribiert die Gene für das einzelsträngige, DNA-bindende Protein DBP, für die adenovirale DNA-Polymerase sowie das terminale Protein pTP, die für die Neusynthese adenoviraler DNA unverzichtbar sind. Aufgrund seiner auf die Vektor-DNA beschränkten Replikationsfähigkeit kann der erfindungsgemäße Vektor eine Stabilität von einem Jahr und mehr erreichen.

Der erfindungsgemäße, in der E1-Region nur teilweise deletierte Vektor wurde mit anderen adenoviralen Vektoren verglichen, denen in der E1-Region längere Nukleotid-Sequenzen entfernt worden waren. So zeigte sich bei einem Vektor, hier Typ "B" genannt, dem die Nukleotide der Positionen 325 bis 3523 entfernt waren, keinerlei Fähigkeit zur Autoreplikation mehr. Weder der erfindungsgemäße Vektor, im folgenden Typ "A" genannt, noch der Vektor Typ B zeigen irgendwelche zytopathischen Wirkungen, noch bilden sie infektiöse virale Partikel. Damit ist erstmalig gezeigt, daß bei einer weniger als üblich ausgedehnten Deletion eines adenoviralen Vektors in der E1-Region die Fähigkeit zur Autoreplikation erhalten bleibt, während die Bildung infektiöser adenoviraler Partikel weiterhin unterbleibt. Dieses Ergebnis ist auch deshalb überraschend, weil in einer erst kürzlich erschienenen wissenschaftlichen Veröffentlichung (6) die Ansicht vertreten wurde, daß die Verweildauer des rekombinaten Adenovirus in vivo nicht von der Vektor DNA-Replikation abhängt. Allerdings wurden in jener Arbeit keine besonders sensitiven Verfahren zur Detektion limitierter Autoreplikation verwendet.

Der erfindungsgemäße adenovirale Vektor kann beliebige transgene DNA-Sequenzen für therapeutisch wirksame Gene aufnehmen. Sehr gut geeignet sind die DNA-Sequenzen der verschiedenen Blutgerinnungsfaktoren, z.B. die Sequenzen der Faktoren IX und VIII.

Das Prinzip eines nicht infektiösen, aber autoreplikativen Adenovektors in der vorstehend beschriebenen Bedeutung kann auch realisiert werden, wenn man von dem in der deutschen Patentanmeldung 198 07 265.1 beschriebenen Transfervektor, im folgenden Minivektor genannt, ausgeht. Werden in dem Minivektor die für die Autoreplikation essentiellen Adenovirus-Gene, nämlich die Gene für das einzelsträngige DNA- bindende Protein DBP, für die adenovirale DNA-Polymerase sowie für das terminale Protein pTP belassen, dann werden dadurch die Vorteile der Autoreplikationsfähigkeit mit denen der minimalen Immunogenität kombiniert. Dieser neue Vektor könnte dann als "autoreplikativer Minivektor" bezeichnet werden.

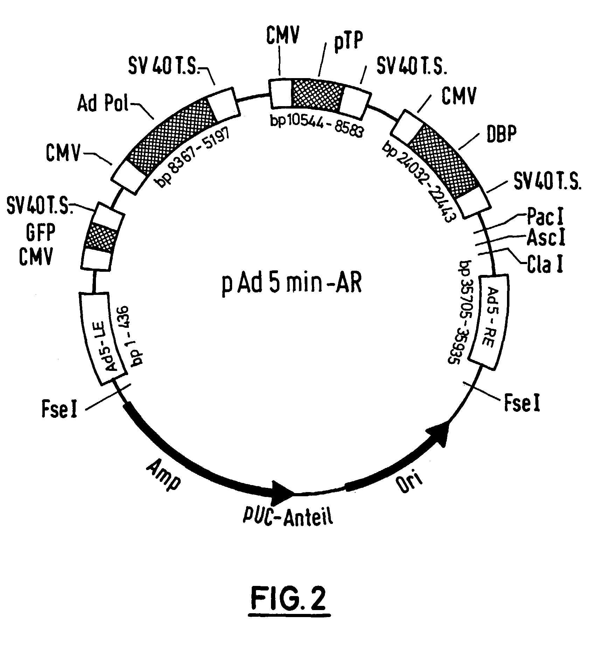

Gegenstand der Erfindung sind deshalb auch rekombinante adenovirale Vektoren dieses Typs, die außer den vorstehend genannten für die Autoreplikation essentiellen Adenovirus-Genen keine anderen natürlichen adenoviralen Proteine mehr exprimieren. Sie sind folgendermaßen aufgebaut (siehe auch Fig.2):
a) Eine erste DNA-Sequenz mit der linken terminalen invertierten Sequenzwiederholung (ITR) und einem Verpackungssignal des Wilddtyp Adenovirus (Serotyp 5);
b) eine Expressionskassette für das Green Fluorescent Protein (GFP), das im Rahmen der Aufreinigung des autoreplikativen Minivektors als Markergen benötigt wird;
c) Expressionskassetten für die Autoreplikationsgene AdPol, DBP, pTP (jeweils unter CMV-Promotor-Kontrolle);
d) Polylinker mit Schnittstellen für Restriktions-endonukleasen, in welche das/die therapeutischen Gene eingebaut werden;
e) eine zweite DNA-Sequenz mit der rechten terminalen invertierten Sequenzwiederholung (ITR) des Wildtyp Adenovirus (Serotyp 5);
f) die ITRs sind von Schnittstellen der Restriktions endonuklease Fsel eingeschlossen, wodurch ein Ausschneiden des adenoviralen Anteils des Transfervektors aus dem pUC19 Trägerplasmid möglich ist.

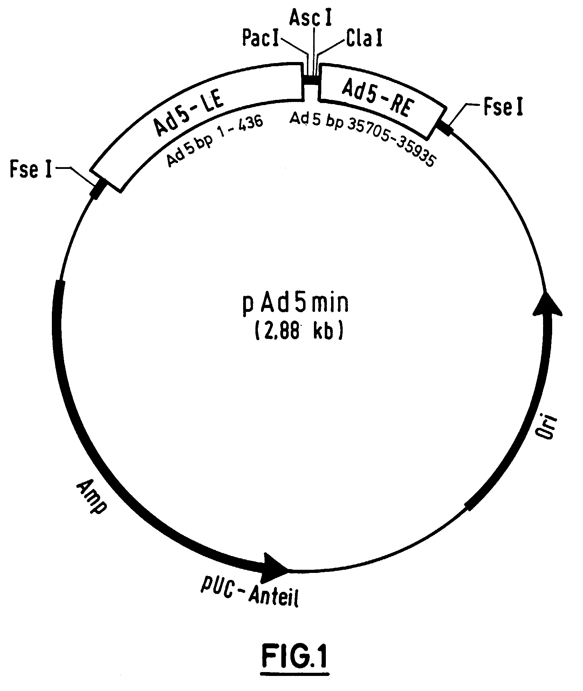

Das in Fig. 1 dargestellte, nicht-autoreplikative Minivektor- plasmid pAd5min gemäß deutscher Patentanmeldung 198 07 265.1 ist das Ausgangsplasmid für diesen Typ autoreplikativer Minivektoren. Dargestellt sind die Sequenzen des Plasmids pUC19, an die über die Schnittstelle FseI zunächst die linke terminale invertierte Sequenzwiederholung und ein Verpackungssignal (ITR und Verpackungssignal= Ad5-LE) mit den Basenpaaren 1 bis mindestens 358 des Wildtyp Adenovirus (Serotyp 5) angefügt sind. Dann folgen die Schnittstellen PacI, AscI und ClaI (multiple cloning site), die mit der DNA-Sequenz der rechten terminalen invertierten Sequenzwiederholung (ITR = Ad5-RE) bestehend aus den Basenpaaren 35.705 bis 35.935 verbunden ist. Anschließend ist erneut eine Fsel-Schnittstelle eingefügt. Dieses Plasmid-Konstrukt erlaubt das Ausschneiden des Ad5-Anteil mittels des Enzyms Fsel. Die entstandene lineare DNA enthält die gleichen Enden wie der lineare Adenovirus-Wildtyp, verlängert auf jeder Seite um eine Base Cytosin.

Dieser Vektor wurde so konstruiert, daß der Einbau von Markergenen, zum Beispiel für das Green Fluorescent Protein (GFP), oder von therapeutischen Genen wie denen für Faktor VIII oder Faktor IX oder zum Beispiel immunmodulierenden Adenovirus-Genen der E3-Region leicht möglicht ist. Die Restriktionsendeonukleasen PacI, AscI und FseI besitzen individuelle Schnittstellen, die jeweils 8 bp lang sind und daher nur sehr selten schneiden. Die Schnittwahrscheinlichkeit beträgt 1 : 4⁸, d.h. sie schneiden durchschnittlich nur alle 65.000 bp einmal. Damit erlauben die PacI- und AscI-Schnittstellen das Einfügen unterschiedlichster Gene, da diese nicht von den Restriktionsendeonukleasen geschnitten werden. ClaI schneidet zwar häufiger, zeichnet sich aber dadurch aus, daß es im Adenovirus nur einmal schneidet. Pacl hat den Vorteil, daß es zur Klonierung des Markergens GFP gut geeignet ist, während AscI und ClaI das wiederholte Aneinanderreihen verschiedener Gene über die gleichen Schnittstellen ermöglichen. Die AscI-Schnittstelle erlaubt die Klonierung eines DNA-Fragments an diese Schnittstelle, wenn das DNA-Fragment in der 5'-Position eine MluI und in der 3'-Position eine AscI-Schnittstelle besitzt, wobei wiederum eine AscI-Schnittstelle entsteht. Ähnlich kann an die ClaI-Schnittstelle kloniert werden. Das seltene Vorkommen von FseI-Schnittstellen in der DNA ermöglicht das Ausschneiden des AdenovirusAnteils zusammen mit den eingefügten Genen aus dem Gesamtplasmid.

In einen derartigen adenoviralen Transfervektor können zwischen die beiden adenoviralen Enden Expressionskassetten mit bis zu etwa 35 kb DNA insertiert werden. Damit kann z.B. die komplette oder auch verkürzte cDNA-Sequenzen der Faktoren VIII oder IX und ggf. weitere Expressionskassetten mit immunmodulierenden und/oder DNA-stabilisierenden Genen in das Viruskonstrukt eingebaut werden.

In Fig. 2 ist ein Beispiel für ein aus Ad5min abgeleitetes, erfindungsgemäßes, autoreplikatives Minivektorplasmid gezeigt, in den weitere Expressionskassetten eingefügt sind, die jeweils mit einem Cytomegalovirus (CMV)-Promotor und einem SV 40 Terminationssignal versehen sind, zwischen denen die für die Autoreplikation erforderlichen adenoviralen Gene (AdPol, DBP, pTP) angeordnet sind. Das Vektorplasmid enthält außer AdPol-, DBP-, pTP-Kassetten noch eine Expressionskassette für GFP, das bei der Aufreinigung des Minivektors eingesetzt wird. Therapeutische Gene werden in die multiple cloning site einkloniert.

Für die Herstellung von rekombinanten Viren des Typs autoreplikativer Minivektor ist eine gleichzeitige Transfektion einer HEK 293-Zellinie mit einem rekombinanten, d.h. ein therapeutisches Gen enthaltenden Minivektorplasmid des Typs pAd5min-AR und einem E1-deletierten Helfervirus (z.B.RR5) oder einem Helferplasmid (zB pJM17) notwendig. Der Transfervektor pAd5min-AR und seine Derivate exprimieren keine adenoviralen Strukturproteine. Deshalb müssen die für die Synthese von Adenoviren notwendigen Strukturproteine über Helfervirus/Helferplasmid in trans zur Verfügung gestellt werden. Nach Produktion der autoreplikativen Minivektor-Viren in Zellkultur werden diese über wiederholte Cäsiumchlorid-Gradienten-Zentrifugationen vom Helfervirus gereinigt, wobei die Expression des GFP aus dem rekombinanten Minivektor als leicht detektierbarer Marker für diesen Minivektor dient.

Die der Erfindung zugrundeliegenden Erkenntnisse sind das Ergebnis der nachfolgend dargestellten Untersuchungen:

### 1. Herstellung eines rekombinanten adenoviralen Vektors

(a) Zur Herstellung eines rekombinanten Vektors vom Typ A wurde die interessierende Fremd-cDNA ausgeschnitten und in das für eukaryotische Zellen geeignete Expressionsplasmid pZS2 eingefügt, das Sequenzen enthielt, die von der adenoviralen 5'-ITR bis zur Nucleotidposition 445 reichen, an die sich ein CMV-Promotor, ein Polylinker, Endsignale für das Gen des bovinen Wachstumshormons (BGH), eine einzige XbaI-Schnittstelle für die in vitro Verknüpfung des linearisierten Plasmids mit dem Adenovirusvektor RR5, eine vom Ad-dl309 abgeleitete Adenovirus-Mutante, aus der die E1-Region zwischen den Nukleotidpositionen 445 - 3333 entfernt ist, und eine weitere einzige XbaI-Schnittstelle stromauf der Deletion anschließen. Der Adenovirusvektor RR5 wurde in menschlichen embryonalen Nierenzellen Stamm 293 vermehrt und nach Isolierung über CsCl-Dichtegradientenzentrifugation mit anschließender Entsalzung über Sephadex7 (Pharmacia AB) G-25 Säulenchromatographie erhalten. Virale DNA wurde aus dieser Viruspräparation isoliert und mit Xba-Restriktionsendonuklease geschnitten, wodurch das Genom in ein großes 3'-terminales und ein kleineres 445 kb 5'-terminales Fragment zerlegt wurde, welches dann von dem großen 3'-XbaI-Fragment mittels Ultrazentrifugieren durch einen 10-40% Sucrose-Gradienten abgetrennt wurde.
   Das rekombinante, mit der interessierenden Fremd-cDNA verknüpfte pZS2 Plasmid wurde nun mit dem großen XbaI Fragment aus RR5 ligiert und in den oben genannten embryonalen Nierenzellen vermehrt, nachdem diese mit dem Ligationsprodukt durch die Calciumphosphat-Methode transfeziert waren. Virale Plaques waren nach einer 12 bis 15-tägigen Zellkultur zu erkennen. Nach einer vollständigen Lysis der virus-enthaltenden Zellkulturen wurde eine Standard-Plaque-Reinigung unter Verwendung von konfluenten 293 Zellkulturen durchgeführt, die mit einer Agarose mit niedrigem Schmelzpunkt (Sigma, Deisenhofen, Bundesrepublik Deutschland) überlagert waren. Nach 5 bis 7 Tagen wurden einzelne Plaques unter der Agaroseschicht sichtbar, die herausgenommen und für eine Polymerasekettenreaktion vorbereitet wurden: das Plaquematerial wurde zusammen mit der Agarose dreimal hintereinander eingefroren und wieder aufgetaut, 30 min bei 37°C in einem Lysispuffer (Ammoniumsulfat 16,6 mM, Tris-HCl pH 8,8 67mM, MgCl₂ 6,7 mM, 2-Mercaptoethanol 5mM, EDTA 6,7 mM, SDS 1,7 mM, Proteinase K 50 µg/ml) inkubiert und dann bei 85°C über einen Zeitraum von 10 min inaktiviert Schließlich wurde die DNA aus dem Lysat durch die übliche Phenol/Chloroform Extraktion isoliert. Unter Verwendung dieser DNA als Sonde wurden rekombinante Plaques, die den interessierenden Vektor enthielten, durch Polymeraseketten-reaktion identifiziert Für die Polymerasekettenreaktion wurden folgende Versuchsbedingungen angewendet: Anfängliche Denaturierung über 5 min bei 94°C, dann 30 Zyklen mit 1 min Denaturierung bei 94°C, 1 min Annealing bei 55-73°C, anschließend 2 min 30 sec (plus 10 sec für jeden zusätzlichen Zyklus) bei 72°C halten, dann noch 10 min stehen lassen. PCR-positive Klone wurden anschließend zwei zusätzlichen Zyklen der Standard-Plaque-Reinigung unterworfen.
(b) Zur Herstellung eines Typ B Vektors wurde ein Plasmid pCMVI durch Klonieren der Expressionkassette des unter Kontrolle des CMV Promotors/Enhancer stehenden Plasmids pCl (Promega, Heidelberg, Bundesrepublik Deutschland) in das adenovirale Transferplasmid pDE1sp1A (Microbix, Toronto, Canada) erzeugt. Dann wurde die interessierende Fremd-cDNA in das Plasmid pCMVI eingeführt Das entstandene Expressions-plasmid wurde dann zusammen mit dem zirkularisierten adenoviralen Genom pJM17 (Microbix) in 293 Zellen übertragen. Virale Plaquen erschienen etwa 10 bis 20 Tage nach der Transfektion. Eine Prüfung mit der Polymerasekettenreaktion auf den rekombinanten Virus wurde wie oben beschrieben durchgeführt.

### 2. Prüfung des adenoviralen Vektors auf cytopathische Wirkungen in den Zielzellen

Konfluente 293 Zellen und EA.hy926 einlagige Zellschichten wurden mit den Autoreplikations-Vektoren RR5 oder Ad5CMVFIX (für Blutgerinnungsfaktor IX) oder mit der Wildtyp-Mutante Ad-dl327 [Multiplizität der Infektion (MOI) = 2] transfeziert. 5 bis 6 Tage nach der Transfektion erschienen auf allen 293 einlagigen Zellschichten Plaques, wie es auch wegen ihrer stabilen Expression von E1 Helferfunktionen zu erwarten war.

Im Gegensatz dazu wurden auch bei verlängerten Inkubationszeiten die einlagigen EA.hy926 Schichten weder durch E1-deletierte RR5-Vektoren noch durch Ad5CMVFIX-Vektoren lysiert, wohl aber durch den Addl327-Vektor. Das zeigt das völlige Fehlen von zytopathischen Wirkungen bei E1-deletierten Autoreplikationsvektoren. Außerdem konnte auch mit dem Standard-LDH-Nachweis bei keinem der untersuchten Vektoren eine Zytotoxizität festgestellt werden.

### 3. Herstellung eines Minivektors mit limitierter Autoreplikationsfähigkeit ausgehend von dem Transfervektor der deutschen Patentanmeldung 198 07 265.1

Ausgangsmaterial war das "leeres" Minivektorplasmid pAd5min der Größe 2,88 kb (Fig.1), das in der deutschen Patentanmeldung 198 07 265.1 beschrieben ist, welches keine adenoviralen Proteine mehr exprimiert (siehe auch: Haemophilia, Vol.4, Number 3, May 1998, page 162, Abstract-No.32). In dieses Minivektorplasmid werden die folgenden adenoviralen Gene, die für die Autoreplikation erforderlich sind, jeweils unter Kontrolle eines CMV-Promotors einkloniert:
1. Adenovirale DNA Polymerase (AdPol), deren Position im Ad5-Genom bei den Nukleotidpositionen 8367 bis 5197 liegt, wobei die Klonierung in Ad5min invers erfolgt.
2. Präterminales Protein (pTP), dessen Position im Ad5-Genom bei den Nukleotidpositionen 10544 bis 8583 liegt, wobei die Klonierung in Ad5min invers erfolgt.
3. Das Single-strand DNA binding protein (DBP), dessen kodierende Sequenz im Adenovirus Typ 5 (Ad5) genom die Nukleotidpositionen 24032 bis 24443 einnimmt (das Gen wird in 3'-5'-Richtung abgelesen). Die Klonierung in Ad5min erfolgt invers zum Ad5 Genom, so daß die Reihenfolge der Sequenzen folgendermaßen lautet: Ad5-Le - CMV-Promotor - DBP-kodierende Sequenz - SV40 Terminationssignal (TS).

Die endgültige Reihenfolge der Sequenzen in der rekombinanten, autoreplikativen Variante von Ad5min (Codename Ad5min-AR = Fig.2) ist wie folgt:
Ad5-LE - CMV-Promotor - Markergen(GFP) - SV40 Terminationssignal (TS) - CMV-Promotor - AdPol-kodierende Sequenz - SV40 Terminationssignal (TS) - CMV-Promotor - pTP-kodierende Sequenz - SV40 Terminationssignal (TS) - CMV-Promotor - DBP-kodierende Sequenz - SV40 Terminationssignal (TS) - Ad5-RE.

### 4. Herstellung des rekombinanten, adenoviralen Vektors für den humanen Blutgerinnungsfaktor IX

### (a) Gewinnung der humanen Factor IX cDNA:

FIX-cDNA wurde mittels der RT-PCR (Reverse-Transcriptase-Polymerasekettenreaktion) kloniert, ausgehend von der aus der menschlichen Leber isolierten Gesamt-RNA. Dazu wurde eine Leberprobe eines männlichen Patienten (45 Jahre) mit normalen hämostatischen Werten, gemessen mit dem RNeasy Kit (Quiagen), verwendet. PolyA*-mRNA wurde aus der Gesamt-RNA gereinigt, wobei das Oligotex Harz (Quiagen) eingesetzt wurde, und für die Synthese einer einsträngigen cDNA verwendet, bei der die AMV Reverse-Transcriptase (Boehringer Mannheim) mit anschließender RT-PCR und thermostabile AmpliTaq DNA Polymerase (Applied Biosystems) benutzt wurden. Das erhaltene Produkt wurde zuerst in das PCR-Script Plasmid (Stratagene) kloniert und mit einem 373A DNA Sequencer (Applied Biosystems) sequenziert.

### (b) Herstellung eines E3-Region positiven Factor IX mit einem CMV Promotor:

Die sequenzierte FIX-cDNA wurde dann herausgeschnitten und in das für eukaryotische Zellen geeignete Expressionsplasmid pZS2 eingefügt, welches eine Adenovirus-Sequenz enthält, die von der adenoviralen 5'-ITR bis zur Nukleotidposition 445 reicht, an die sich der CMV-Promotor, ein Polylinker, die Terminationssignale des bovinen Wachtumshormongens und eine einzige XbaI Schnittstelle anschließen, welche dazu benutzt wird, das linearisierte FIX/pZS2 Plasmid in vitro in den adenoviralen Vektor RR5 einzuklonieren. RR5 ist ein von Ad-dl309 abgeleitetes, adenovirales Genom mit einer Deletion der E1-Region (von der Nukleotid-Position 445 bis 3333), durch die der Vektor die Fähigkeit zur Replikation verloren hat. RR5 hat jedoch die E3-Region des Adenovirus Typ 5 (Ad5) zurückbehalten. RR5 wurde in humanen embryonalen 293 Nierenzellen vermehrt und dann durch eine CsCl-Dichtegradientenzentrifugation isoliert, an die sich ein Aussalzen auf Sephadex 25 Säulen (Pharmacia) anschloß. Aus dieser gereinigten Virus-Präparation wurde virale, genomische DNA isoliert und in vitro mit XbaI geschnitten. Dadurch wurde ein 445 kb umfassendes, endständiges XbaI-Fragment des Genoms freigesetzt, welches dann vom dem großen 3'XbaI-Fragment mittels Ultrazentrifugieren durch einen 10 bis 40% Sucrosegradienten abgetrennt wurde. Das Verknüpfungsprodukt des langen XbaI-Fragments des RR5 mit dem rekombinanten FIX/pZS2 Expressionsplasmid wurde mit der Calciumphosphat-Methode in 293 Zellen transfeziert. Virale Plaquen waren in der Zellkultur nach 12 bis 15 Tagen sichtbar. Nach vollständiger Lysis der Viren enthaltenden Zellkulturen wurde eine Standard-Plaque-Reinigung des lysierten Materials durchgeführt, bei der eine konfluente 293 Zellkultur eingesetzt wurde, die mit einer niedrig schmelzenden Agarose (Sigma) überdeckt war. Nach 5 bis 7 Tagen wurden einzelne Plaques unter der Agaroseschicht sichtbar, die herausgenommen und für eine Polymerasekettenreaktion weiterverarbeitet wurden: Das Plaque-Material plus Agarose wurde dreimal hintereinander gefroren und wieder aufgetaucht, dann 30 min bei 37°C in einem Lysis-Puffer inkubiert und schließlich über einen Zeitraum von 10 min bei 85°C inaktiviert. Die DNA wurde dann aus dem Lysat durch die übliche Phenol/Chloroform Extraktion isoliert. Die gewonnene DNA wurde als Sonde verwendet und die rekombinanten Plaques, welche den Vektor Ad5E3+FIX enthielten, durch Polymerasekettenreaktion identifiziert, wobei die folgenden Primer-Paare eingesetzt wurden:
- a. SEQ ID NO: 1:: 5'-CCG TGC TGA GGC TGT TTT TCC -3'
(FIX-code)
- b. SEQ ID NO: 2:: 5'-CAG CTC CTC GGT CAC ATC CA-3'
(Ad5-anti)
- c. SEQ ID NO: 3:: 5'-TCT CTC AAG GTT CCC TGA AAC-3'
(FIX-anti)
- d. SEQ ID NO: 4:: 5'-TGA TAA TGA GGG GGT GGA GTT TGT -3'
(Ad5-code)

Dabei wurden folgende Bedingungen für die Polymerasekettenreaktion eingehalten: Die anfängliche Denaturierung wurde über einen Zeitraum von 5 min bei 94°C durchgeführt; dann erfolgten 30 Zyklen mit jeweils 1 min Denaturierung bei 94°C, dann 1 min Annealing bei 65°C; anschließend wurde für 2 min 30 sec (plus 10 sec für jeden Zyklus) eine Temperatur von 72°C eingehalten, die dann noch weitere 10 min beibehalten wurde. PCR-positive Klone wurden dann gereinigt und zwei weiteren Zyklen einer Standard-Plaque-Reinigung unterworfen und als Ad5E3+FIX Vektor bezeichnet.

### Literaturverzeichnis:

1. Byun, J., S.-H. Kim, S.S. Yu, P. Robbins, J. Yim, and S. Kim. 1996. Analysis of the relative level of gene expression from different retroviral vectors used for gene therapy. *Gene Therapy.* 3:780-788.
2. Lynch, C., P. Hara, J. Leonard, J. Williams, R. Dean, and R. Geary. 1997. Adeno-associated virus vectors for vascular gene delivery. *Circulation Research.* 80:497-505.
3. Rubenstein, R., U. McVeigh, T. Flotte, W. Guggino, and P. Zeitlin. 1997. CFTR gene transduction in neonatal rabbits using an adeno-associated virus (AAV) vector. *Gene Therapy*. 4.(5):384-392.
4. Li, Q., M. Kay, M. Finegold, L. Stratford-Perricaudet, and S. Woo. 1993. Assessment of recombinant adenoviral vectors for hepatic gene therapy. *Human Gene Therapy.*4:403-409.
5. Miller, R., and D. Curiel. 1996. Towards the use of replicative adenoviral vectors for cancer gene therapy. *Gene Therapy.* 3(7):557-559.
6. Nelson I. E., M. A. Kay, 1997. Persistence of Recombinant Virus in vivo is not dependent on Vector DNA Replication. J. of Virology Nov. 1997 : P. 8902-8907.

## Patentansprüche

1. Rekombinanter, adenoviraler Vektor, **dadurch gekennzeichnet**, dass er nach nur teilweiser Deletion der E1-Region noch die Fähigkeit zur Autoreplikation in vivo besitzt, aber keine infektiösen, adenoviralen Partikel mehr bilden kann.

2. Adenoviraler Vektor nach Anspruch 1, **dadurch gekennzeichnet**, dass er eine transgene DNA-Sequenz für ein therapeutisch wirksames Gen trägt.

3. Adenoviraler Vektor nach den Ansprüchen 1 und 2, **dadurch gekennzeichnet,** dass er zur Autoreplikation in einem Maße befähigt ist, der in etwa dem natürlichen Abbau des Vektors entspricht.

4. Adenoviraler Vektor auf der Basis des Adenovirus Typ5-Genoms nach den Ansprüchen 1 bis 3, **dadurch gekennzeichnet,** dass seine E1A-Region vollständig entfernt ist, während das E1B-55k Gen zwischen den Nukleotidpositionen 3334 bis 3508 sowie das komplette E1B-14k Gen beginnend mit der Nukleotidposition 3608 erhalten geblieben sind.

5. Adenoviraler Vektor nach Anspruch 1, **dadurch gekennzeichnet**, dass er auf der Basis des Vektor-Plasmids Ad5 min außer dessen viralen ITR-Sequenzen und dem Verpackungssignal noch Expressionskassetten für das Autoreplikations-relevante, DNA bindende Protein DBP, für die adenovirale DNA-Polymerase sowie für das präterminale Protein pTP trägt.

6. Arzneizubereitung zum Einsatz in der Gentherapie, **dadurch gekennzeichnet,** dass sie einen adenoviralen Vektor der Ansprüche 1 bis 5 enthält.

7. Verfahren zur Herstellung von adenoviralen Vektoren der Ansprüchen 1 bis 4, **dadurch gekennzeichnet,** dass die E1-Region partiell, nicht jedoch die Gene für das einzeisträngige, DNA-bindende Protein DBP, für die adenovirale DNA-Polymerase sowie für das terminale Protein pTP entfernt wird, wodurch die Fähigkeit zur Autoreplikation noch erhalten bleibt, aber keine infektiösen, viralen Partikel mehr gebildet werden können.

8. Verfahren nach Anspruch 7, **dadurch gekennzeichnet,** dass außer den für die Autoreplikation unverzichtbaren Genen alle anderen, für die Expression natürlicher adenoviraler Proteine benötigten Gene entfernt werden.

9. Verwendung adenoviraler Vektoren der Ansprüche 1 bis 5 in der somatischen Gentherapie.
